# EUROPÄISCHE PATENTSCHRIFT

(11) **EP 1 129 745 B1**
(45) Veröffentlichungstag und Bekanntmachung des Hinweises auf die Patenterteilung: **20.07.2005**
(21) Anmeldenummer: 01250066.6
(22) Anmeldetag: 01.03.2001
(51) Int. Cl.: A61N 1/05

(54) **Elektrodenanordnung**
Electrode device
Structure d'électrode

(30) Priorität: 02.03.2000 DE 10011572
(43) Veröffentlichungstag der Anmeldung: 05.09.2001
(73) Patentinhaber: BIOTRONIK Mess- und Therapiegeräte GmbH & Co Ingenieurbüro Berlin, 12359 Berlin (DE)
(72) Erfinder: Audoglio, Roberto, 27010 Linarolo (IT)
(74) Vertreter: Eisenführ, Speiser & Partner

(56) Entgegenhaltungen:
- WO-A-99/53993
- US-A- 5 443 492
- US-A- 5 571 162
- US-A- 5 693 081

## Beschreibung

Die Erfindung betrifft eine Elektrodenanordnung mit einer Elektrodenleitung, welche mindestens ein erstes Befestigungselement trägt, das zum Befestigen der Elektrodenleitung an Körpergewebe, insbesondere dem Myokard eines Herzens ausgebildet und mit Abstand zum distalen Ende der Elektrodenleitung angeordnet ist.

Die Erfindung betrifft insbesondere eine Elektrodenleitung mit Elektroden zur Stimulation des Myorkards eines menschlichen Herzens oder zum Aufnehmen elektrischer Signale von einem menschlichen Herz. Derartige Elektrodenleitungen werden vor allem in Verbindung mit einem implantierten Herzschrittmacher verwendet, der über die Elektroden der Elektrodenleitung künstlich stimuliert.

Von besonderem Interesse ist unter anderem die Stimulation des Septums zwischen linkem und rechtem Ventrikel des Herzens. Elektrodenanordnungen zur Stimulation des Septums sind beispielsweise aus der DE 195 46 941, der US 5,728,140, der US 5,683,447, der US 5,476,500 und der US 5,693,081 bekannt. Keine dieser Elektrodenleitungen erlaubt in befriedigender Weise eine biphasische Stimulation des oberen Septums. Auch ist die Verankerung der Elektrodenleitung im Septum bei den bekannten Elektrodenanordnungen nicht befriedigend gelöst. US 5,693,081 offenbart die Merkmale des Oberbegriffs des Anspruchs 1.

Vor dem Hintergrund des Standes der Technik liegt der Erfindung die Aufgabe zu Grunde, eine alternative Elektrodenanordnung zu bieten, die beschriebenen Nachteile des Standes der Technik möglichst vermeidet.

Gelöst wird diese Aufgabe erfindungsgemäß durch die Merkmale des charakterisierenden Teils des Anspruchs 1.

Eine derartige Elektrodenanordnung kommt in vorteilhafter Weise ohne relativ zur Elektrodenleitung bewegbare Befestigungsmittel aus, wie sie aus den US-Patentschriften 5,476,500, 5,683,447 und 5,693,081 bekannt sind.

Erfindungsgemäß ist das erste Befestigungselement als Helixwendel ausgebildet, die mit der Elektrodenleitung starr verbunden ist, so daß die Helixwendel die Elektrodenleitung im wesentlichen koaxial mit Abstand zur Elektrodenleitung umschlingt. Das erste Befestigungselement ist somit korkenzieherartig ausgebildet und starr mit der Elektrodenleitung verbunden. Das freie Ende des Befestigungselementes hakt durch Drehen der Elektrodenleitung um deren Längsachse in benachbartes Körpergewebe ein und sorgt somit für eine sichere Befestigung der Elektrodenleitung an dem Körpergewebe. Dies ermöglicht insbesondere eine aktive Fixierung an dem Septum eines Herzens. Die Gänge der Wendel haben dabei vorzugsweise einen gleichbleibenden Abstand voneinander, wie dies auch die Gänge eines Korkenziehers haben. Außerdem ist das freie Ende des Befestigungselementes vorzugsweise angespitzt.

Das erste Befestigungsmittel ist vorzugsweise mit seinem distalen Ende an der Elektrodenleitung befestigt, so daß das freie Ende des ersten Befestigungsmittels dem proximalen Ende der Elektrodenleitung näher ist. Zusammen mit einem korkenzieherartig gewundenen zweiten Befestigungselement mit entgegengesetztem Drehsinn am distalen Ende der Elektrodenleitung kann, wie weiter unten beschrieben, eine Belastung der Elektrodenleitung bei kontraktierendem Myokard vermieden werden.

In einer ebenso bevorzugten alternativen Ausführungsform weist das erste Befestigungselement ein zweites freies Ende auf, welches bezogen auf die Elektrodenleitung die gleiche tangentiale Ausrichtung wie das erste freie Ende besitzt, aber eine entgegengesetzte axiale Ausrichtung, wobei das erste Befestigungselement zwischen beiden freien Enden an der Elektrodenleitung befestigt ist. Eine derartige mittige Befestigung des ersten Befestigungselemntes an der Elektrodenleitung mit zwei freien Enden besitzt wegen der gegenlaufigen Drehsinne der beiden resultierenden Helixabschnitte den Vorteil, daß sich beim Einschrauben der Helixabschnitte in benachbartes Gewebe eine gewisse Verspannung der beiden freien Enden gegeneinander einstellt. Diese Verspannung führt zu einer sicheren Fixierung der ansonsten eher durch unbeabsichtigte Drehbewegungen lösbaren Befestigung.

Die Elektrodenanordnung trägt erfindungsgemäß ein zweites Befestigungselement an ihrem distalen Ende. Eine derartige Elektrodenanordnung kann an zwei Stellen, nämlich an ihrem distalen Ende und mit Abstand davon aktiv am Körpergewebe befestigt werden, so daß die beiden Befestigungselemente auch bezüglich des Körpergewebes eine feste Position zueinander einnehmen.

Dabei ist auch das zweite Befestigungselement nach Korkenzieherart als Helixwendel mit einem freien Ende ausgebildet, wobei das zweite Befestigungselement einen dem ersten Befestigungselement entgegengesetzten Drehsinn besitzt. Wenn auch das zweite Befestigungselement starr mit der Elektrodenleitung verbunden ist, kann die Elektrodenleitung von ihrem proximalen Ende her um ihre Längsachse gedreht werden, um auf diese Weise beide Befestigungselemente im gleichen Sinne in benachbartes Körpergewebe eindringen zu lassen. Infolge des entgegengesetzten Drehsinns der beiden Befestigungselemente und der hiermit in unmittelbarem Zusammenhang stehenden Maßnahme, das erste Befestigungselement mit seinem distalen Ende an der Elektrodenleitung zu befestigen, wird das Myokard beim Einschrauben der Befestigungselemente zwischen beiden gestaucht. Auf diese Weise werden hohe Belastungen der Elektrodenleitung im Falle der Kontraktion des Myokards vermieden.

Anstelle das zweite Befestigungselement derart starr mit der Elektodenleitung zu verbinden, daß es sich wie das erste Befestigungselement mit der Elektrodenleitung mitdreht, kann auch ein Antrieb für das zweite Befestigungselement vorgesehen sein, der es erlaubt, das zweite Befestigungselement unabhängig von der Elektrodenleitung um deren Längsachse drehend anzutreiben, und zwar vorzugsweise vom proximalen Ende der Elektrodenleitung her. Vorzugsweise ist das erste Befestigungselement mit der Elektrodenleitung verbunden, während das zweite Befestigungselemnt durch einen in der Elektrodenleitung geführten Mandrin gegenüber der Elektrodenleitung drehbar ist. Diese Art des Antriebs ist für aktiv fixierbare Schraubelektroden am distalen Ende der Elektrodenleitung an sich bekannt.

Besonders bevorzugt ist eine Elektrodenanordnung, welche eine erste Elektrode mit einer elektrisch leitenden Oberfläche auf der dem freien Ende des ersten Befestigungselementes benachbarten Oberfläche der Elektrodenleitung aufweist. Durch diese Anordnung der ersten Elektrode relativ zum ersten Befestigungslelement wird ein intensiver Kontakt der ersten Elektrode mit dem Körpergewebe auf Dauer sichergestellt. Das erste Befestigungselement ist dabei vorzugsweise mit der ersten Elektrode elektrisch leitend verbunden und wird auf diese Weise selbst zum Bestandteil der ersten Elektrode. Die erste Elektrode wird im übrigen vorzugsweise von der von dem ersten Befestigungselement umschlungenen Oberfläche der Elektrodenleitung gebildet.

Die Elektrodenanordnung weist vorzugsweise mindestens eine zweite Elektrode an ihrem distalen Ende auf. Eine derartige zweite Elektrode insbesondere in unmittelbarer Nachbarschaft zu dem zweiten Befestigungselement, welches vorzugsweise Teil der zweiten Elektrode ist, erlaubt die Abgabe bipolarer, insbesondere biphasischer Stimulationsimpulse an das Körpergewebe. Die biphasischen Stimulationsimpulse können sich dabei zeitlich überlappen.

Besonders bevorzugt ist eine Elektrodenanordnung, welche genau zwei Befestigungselemente umfaßt, die als korkenzieherartige Helixwendeln ausgebildet, jeweils mit einem freien, zum Eindringen in Gewebe ausgebildeten Ende versehen und zum einen am distalen Ende der Elektrodenleitung und zum anderen entfernt hiervon angeordnet sind, sowie mindestens zwei Elektroden mit elektrisch leitender Oberfläche, die jeweils in unmittelbarer Nachbarschaft zu den Befestigungsmitteln angeordnet sind. Eine derartige Elektrodenanordnung erlaubt in vorteilhafter Weise das biphasische Stimulieren des hohen Septums eines Herzens.

Die Elektrodenleitung umfaßt vorzugsweise weiterhin zwei koaxiale, elektrisch leitende Wendeln, welche zueinander und zum Äußeren der Elektrodenleitung hin elektrisch isoliert sind und von denen die äußere Wendel die vom distalen Ende der Elektrodenleitung entfernte erste Elektrode kontaktiert während die innere Wendel die Elektrode am distalen Ende der Elektrodenleitung kontaktiert. Eine derartige Elektrodenleitung weist eine hohe Flexibilität auf, ohne daß die Gefahr des Bruches der elektrisch leitenden Wendeln besteht. Außerdem stellt eine derartige Elektrodenleitung eine sichere Kontaktierung der Elektroden dar.

Die Elektrodenleitung weist außerdem vorzugsweise Steuermittel auf, mit denen das distale Ende in an sich bekannter Weise in die bilaterale Richtung ausgelenkt werden kann. Dies erlaubt ein gezieltes Führen der Elektrodenleitung zu ihrem Zielort. Derartige Steuermittel sind beispielsweise aus den US-Patenten 5,254,088, 5,364,351, 5,376,109 und 5,423,884 sowie der EP 0 667 126 und der DE 35 07 119 bekannt.

Die Erfindung soll nun anhand eines Ausführungsbeispiels mit Hilfe der Figuren naher erläutert werden.
- Fig. 1: eine Außenansicht des distalen Bereichs einer erfindungsgemäßen Elektrodenleitung in vereinfachter Darstellung;
- Fig. 2a: eine Schnittdarstellung durch die Elektrodenleitung aus Figur 1 im Bereich eines vom distalen Ende der Elektrodenleitung entfernten Befestigungselementes;
- Fig. 2b: eine alternative Ausführungsform des Befestigungselementes aus Fig. 2a;
- Fig. 3: eine Darstellung eines menschlichen Herzens im Schnitt mit plazierter Elektrodenleitung.

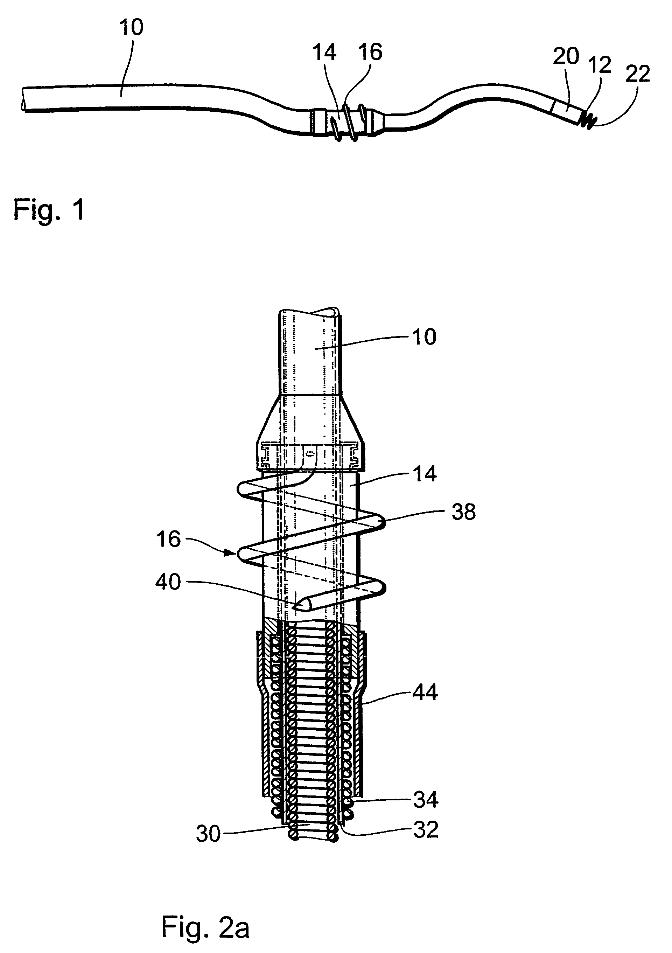

Figur 1 zeigt denjenigen Teil einer Elektrodenleitung 10, der an deren distales Ende 12 anschließt. Mit einem Abstand vom distalen Ende 12 der Elektrodenleitung 10 ist eine erste Elektrode 14 sowie ein erstes Befestigungselement 16 vorgesehen. Das erste Befestigungselement 16 ist an der Elektrodenleitung 10 starr befestigt und umschlingt die Elektrodenleitung 10 nach Korkenzieherart mit einem Abstand zu der Elektrodenleitung 10. Der umschlungene Bereich der Elektrodenleitung 10 ist als erste Elektrode 14 ausgebildet und besitzt eine elektrisch leitende Oberfläche.

Diese steht mit dem ersten Befestigungselement 16 in elektrischem Kontakt, so daß das erste Befestigungselement 16 Teil der ersten Elektrode 14 ist.

Das erste Befestigungselement 16 ist am distalen Ende der ersten Elektrode 14 befestigt, so daß sich ein angespitztes freies Ende 40 des ersten Befestigungselementes 16 in der Nähe des proximalen Endes der ersten Elektrode 14 befindet. Diese Anordnung hat den Vorteil, das Probleme wie ein Verhaken des freien Endes 40 des ersten Befestigungselementes 16 beim Einsetzen der Elektrodenleitung 10 beispielsweise in ein menschliches Herz vermieden werden.

Am distalen Ende 12 der Elektrodenleitung 10 ist eine zweite Elektrode 20 als Tipelektrode vorgesehen. Außerdem ist am distalen Ende 12 ein zweites Befestigungselement 22 befestigt, welches ebenfalls von einem korkenzieherartig geformten Metalldraht gebildet wird, dessen freies Ende zur aktiven Befestigung in einem Herzen in dessen Myokard eingeschraubt werden kann. Dazu wird die gesamte Elektrodenleitung 10 um ihre Längsachse gedreht. Auch das zweite Befestigungselement 22 ist mit der zweiten Elektrode 20 derart verbunden, daß das zweite Befestigungselement 22 zum Bestandteil der zweiten Elektrode wird. Die zweite Elektrode 20 wird im übrigen von einer elektrisch leitenden Oberfläche der Elektrodenleitung 10 an deren distalen Ende 12 gebildet.

Figur 2a zeigt den Bereich der ersten Elektrode 14 und des ersten Befestigungselementes 16 der Elektrodenleitung 10 im Detail. Als eines der Grundbestandteile der Elektrodenleitung 10 ist eine innere Drahtwendel 30 zu erkennen, die von einer schlauchartigen Silikonisolierung 32 umgeben ist und die zu der Tipelektrode 20 führt und diese elektrisch kontaktiert.

Ausgehend vom proximalen Ende der Elektrodenleitung 10 bis hin zur ersten Elektrode 14 ist die Silikonisolierung 32 von einer zweiten Drahtwendel 34 umgeben, die innere Drahtwendel 30 und die schlauchartige Silikonisolierung 32 koaxial umgibt. Die äußere Drahtwendel 34 kontaktiert an ihrem distalen Ende die erste Elektrode 14, die von einer Metallhülse 36 gebildet wird, welche die innere Drahtwendel 30 und die schlauchartige Silikonisolierung 32 koaxial umschließt.

Die erste Elektrode 14 bildende Metallhülse 36 ist mit einem Ende einer korkenzieherartig gewundenen Metallhelix 38 fest, beispielsweise durch Schweißen, mit dem distalen Ende der Metallhülse 36 verbunden. Die korkenzieherartige Metallhelix 38 bildet das erste Befestigungselement 16 und ist so geformt, daß die Wendeln der Metallhelix sich mit einem Abstand voneinander und zu der Metallhülse 36 um diese winden. Die Metallhelix 38 besitzt ein im wesentlichen tangential ausgerichtetes, frei in den Raum ragendes, angespitztes freies Ende 40, welches durch Drehen der gesamten Elektrodenleitung 10 um ihre Längsachse in benachbartes Körpergewebe eindringen kann. Das angespitzte freie Ende 40 befindet sich in der Nähe des proximalen Endes der ersten Elektrode 14.

Die Metallhelix 38 ist so ausgeführt, daß das freie Ende 40 durch Drehen der Elektrodenleitung 10 im Gegenuhrzeigersinn in benachbartes Myokard eindringt und auf diese Weise das Myokard in einen engen Kontakt mit der ersten Elektrode 14 bringt. Dazu sind beispielsweise zweieinhalb Umdrehungen der Elektrodenleitung erforderlich. Die Metallhelix 38 besitzt entsprechend zwei Gänge und erstreckt sich über etwa 80% der Länge der ersten Elektrode 14. Die gewählte Gestaltung und Befestigung der Metallhelix 38 hat weiterhin die Wirkung, daß das Myokard beim Einschrauben des ersten Befestigungselementes 16 in Richtung der Tipelektrode 22 geschoben wird, daß das, wenn man so will, Myokard zwischen den beiden Elektroden etwas gestaucht wird. Dies vermindert eine Belastung der Elektroden und der Elektrodenleitung während der Kontraktion des Myokards.

Zum Entfernen der Elektrodenleitung braucht diese nur im Uhrzeigersinn um ihre eigene Achse gedreht zu werden, um die beiden Befestigungsmittel 16 und 22 aus dem Myokard zu schrauben.

Die äußere Drahtwendel 34 ist von einer äußeren schlauchartigen Silikonisolierung 44 umgeben. Damit besitzt die Elektrodenanordnung genau zwei nach außen elektrisch wirksame Bestandteile, die zum einen von der Tipelektrode 20 und dem zweiten Befestigungselement 22 gebildet werden und zum anderen von der Metallhülse 36 und der Metallhelix 38.

Nicht dargestellt sind Steuer- oder Führungsdrähte im Inneren der Elektrodenleitung, die in bekannter Weise dem Steuern und Führen der Elektrodenleitung durch laterales Auslenken des distalen Endes 12 der Elektrodenleitung dienen.

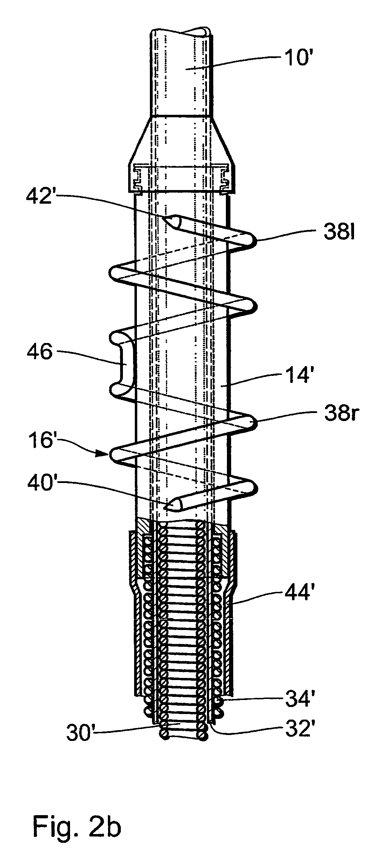

Die Figur 2b zeigt eine Ausführungsform 16' des ersten Befestigungselementes, die sich von dem Befestigungselement 16 aus Figur 2a dadurch unterscheidet, daß das alternative Befestigungselement 16'2 angespitzte freie Enden 40' und 42' aufweist. Die beiden freien Enden 40' und 42' befinden sich am Ende zweier gegenläufiger Metallhelices 38r und 38l, von denen die Metallhelix 38r rechtsdrehend ist, während die Metallhelix 38l linksdrehend ist. Die beiden Metallhelices 38r und 38l gehen von einem mittigen Abschnitt 46 des Befestigungselementes 16' aus, der zur Befestigung des ersten Befestigungselementes 16' in der Elektrode 14' mit dieser verschweißt ist. Das erste Befestigungselement 16' ist somit mittig zwischen seinen beiden freien Enden 40' und 42' mit der Elektrode 14' verbunden. Wenn man davon absieht, daß die Metallhelix 381 linksläufig ist, entsprechen die Metallhelices 38l und 38r der Metallhelix 38 aus Figur 2a. Beim Einschrauben der Metallhelices 38l und 38r in benachbartes Körpergewebe verspannen sich die Metallhelices 38l und 38r gegeneinander und stellen so eine sichere Fixierung des ersten Befestigungselementes 16' in dem Körpergewebe her.

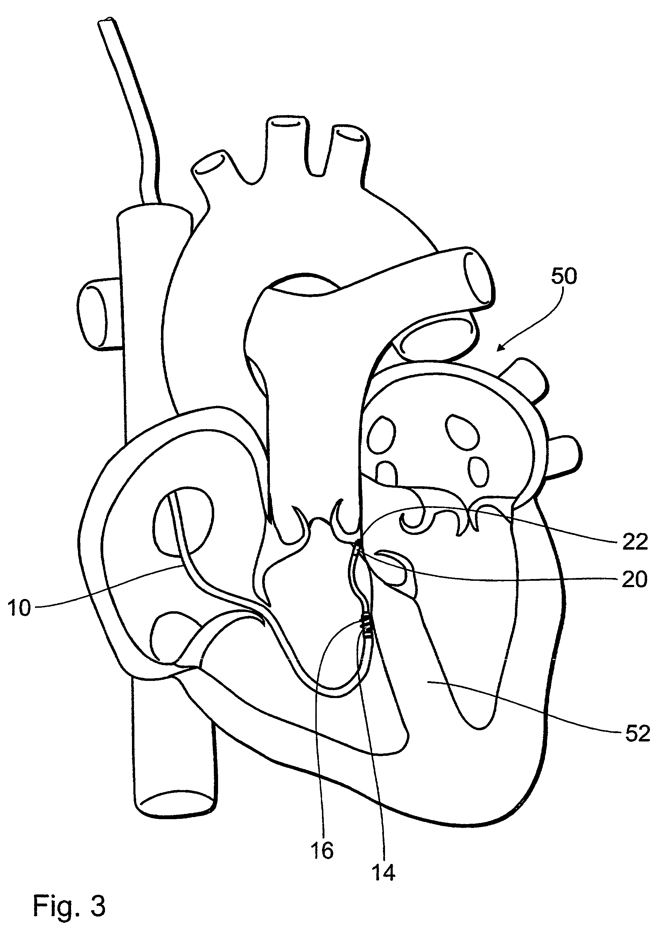

Figur 3 zeigt ein menschliches Herz 50 in der Schnittdarstellung, in welches die Elektrodenleitung 10 mit der ersten Elektrode 14 und der zweiten Elektrode 20 derart eingeführt ist, daß die Tipelektrode 20 am hohen Septum 52 anliegt, und die erste Elektrode 14 ebenfalls am Septum 52 mit vorbestimmtem Abstand zur zweiten Elektrode 20. Beide Befestigungselemente 16 und 22 sind durch axiales Drehen der Elektrodenleitung 10 um ihre Längsachse in das Septum 52 eingeschraubt.

Besonderer Vorzug der abgebildeten Anordnung ist es, eine biphasische Stimulation des hohen Septums mit sich zeitlich überlappenden Impulsen zu ermöglichen, die eine mögliche Alternative zu einer biventrikularen Stimulation für Patienten mit verlängerter interventrikulärer Überleitungszeit ist.

Dazu ist die Elektrodenleitung an ihrem proximalen Ende mit einem an sich bekannten implantierbaren Herzschrittmacher verbunden, der die innere und die äußere Wendel 30 und 34 separat kontaktiert, so daß an die erste Elektrode 14 und die zweite Elektrode 20 unabhängig voneinander elektrische Impulse abgegeben werden können.

Die spezielle Ausgestaltung der beiden Befestigungselemente 16 und 22 erlauben eine aktive Fixierung der Elektrodenleitung an zwei Stellen des Septums 52, bei der das distale Ende 12 der Elektrodenleitung 10 unmittelbar am Stamm der rechten Lungenarterie im hohen Septum befestigt ist, während das erste Befestigungselement 16 etwas entfernt hiervon aber immer noch in das hohe Septum eingreift. Durch die der Figur 3 entnehmbare Anordnung der ersten und zweiten Elektrode 14 und 20 sowie des ersten und zweiten Befestigungselementes 16 und 22 ist sowohl die Gestaltung der Befestigungselemente 16 und 22 als auch der Abstand der Elektroden 14 und 20 sowie der entsprechenden Befestigungselemente 16 und 22 auf der Elektrodenleitung 10 vorgegeben.

Versuche haben ergeben, daß durch eine derartige Anordnung eine doppelte Depolarisierungsfront erzeugt werden kann, die den gesamten Bereich des Septums 52 des Herzens erfaßt, wenn sowohl kathodische als auch anodische Impulse, also zwei gegenphasige Impulse in einen begrenzten, circa zwei Zentimeter großen, unmittelbar an den Eintritt der Pulmunararterie ins Ventrikel angrenzenden Bereich des interventrikulären Septums abgegeben werden. Die von der kathodischen Elektrode ausgehende Depolarisierungsfront wandert dabei von der Oberfläche zum Inneren des septalen Myokards, während die von der anodisch angesteuerten Elektrode ausgehende Depolarisierungsfront vom Inneren des Myokards zu dessen Oberfläche wandert. Die Depolarisierung folgt dabei den natürlichen Überleitungswegen, welche innerhalb des Septums verlaufen, so daß eine Verzögerung der interventrikulären Aktivierung vermieden wird, wie sie bei der üblichen Stimulation beim Apex des rechten Ventrikels vorkommt. Auf diese Weise wird die naturliche physiologische Dynamik der Kontraktion des Herzens wiederhergestellt.

Wichtig ist dabei der durch die zuvor beschriebene Ausführung der Elektrode sichergestellte intensive Kontakt der Elektroden mit dem Myokard. Dieser wird durch die aktive Fixierung mit Hilfe der beschriebenen Befestigungsmittel ermöglicht, die beide jeweils elektrisch leitend ausgebildet und leitend mit den Elektroden verbunden sind, so daß sie selbst zum Bestandteil der Elektroden werden.

## Patentansprüche

1. Elektrodenanordnung mit einer Elektrodenleitung (10), welche mindestens ein erstes Befestigungselement (16) und ein zweites Befestigungselement (22) trägt, welche zum Befestigen der Elektrodenleitung an Körpergewebe, insbesondere dem Myokard eines Herzens ausgebildet sind,
wobei das erste Befestigungselement (16) mit Abstand zum distalen Ende (12) der Elektrodenleitung (10) und das zweite Befestigungselement (22) am distalen Ende (12) der Elektrodenleitung (10) angeordnet ist,
**dadurch gekennzeichnet,**
**dass** das erste Befestigungselement (16) wenigstens einen helixwendelartig gewundenen Abschnitt aufweist, welcher mit der Elektrodenleitung (10)starr verbunden ist, so dass der helixwendelartig gewundene Abschnitt die Elektrodenleitung (10) im wesentlichen koaxial mit Abstand zur Elektrodenleitung (10) umschlingt und ein in proximale Richtung weisendes freies Ende aufweist, welches eine Erstreckungskomponente in bezüglich der Elektrodenleitung (10) tangentialer Richtung aufweist und so ausgebildet ist, daß das erste Befestigungselement (16) durch eine Drehbewegung der Elektrodenleitung (10) um deren Längsachse im Einsatzfall benachbartes Körpergewebe eindringt,
und **dass** das zweite Befestigungselement (22) nach Korkenzieherart als Helixwendel mit einem freien Ende (40) ausgebildet ist und der Drehsinn des zweiten Befestigungselementes (22) dem des ersten Befestigungselementes entgegengesetzt ist.

2. Elektrodenanordnung nach Anspruch 1, **dadurch gekennzeichnet, daß** die Gänge der Wendel des helixwendelartig gewundenen Abschnitts des ersten Befestigungselements (16) in Längsrichtung der Elektrodenleitung (10) voneinander beabstandet sind.

3. Elektrodenanordnung nach einem der Ansprüche 1 bis 2, **dadurch gekennzeichnet, dass** das freie erste Ende des ersten Befestigungselementes (40) angespitzt ist.

4. Elektrodenanordnung nach einem der Ansprüche 1 bis 3, **dadurch gekennzeichnet, dass** das erste Befestigungselement (16) ein zweites freies Ende aufweist, welches bezogen auf die Elektrodenleitung (10) die gleiche tangentiale Ausrichtung wie das erste freie Ende besitzt, aber eine entgegengesetzte axiale Ausnchtung, und dass das erste Befestigungselement (16) zwischen beiden freien Enden an der Elektrodenleitung (10) befestigt ist.

5. Elektrodenanordnung nach einem der Ansprüche 1 bis 4, **dadurch gekennzeichnet, dass** das zweite Befestigungselement (22) derart mit der Elektrodenleitung (10) verbunden ist, dass es zusammen mit der Elektrodenleitung durch Drehen der Elektrodenleitung um deren Längsachse drehend antreibbar ist.

6. Elektrodenanordnung nach Anspruch 4, **gekennzeichnet durch** einen Antrieb, der mit dem zweiten Befestigungselement (22) verbunden und derart ausgebildet ist, dass das zweite Befestigungselement (22) unabhängig von der Elektrodenleitung (10) um deren Längsachse drehend antreibbar ist.

7. Elektrodenanordnung nach Anspruch 6, **dadurch gekennzeichnet, dass** der Antneb derart ausgebildet ist, dass das zweite Befestigungselement (22) vom proximalen Ende der Elektrodenleitung (10) her relativ zur übrigen Elektrodenleitung (10) drehend antreibbar ist.

8. Elektrodenanordnung nach einem der Ansprüche 1 bis 7, **gekennzeichnet durch** eine erste Elektrode (14) mit einer elektrisch leitenden Oberfläche auf der dem freien Ende (40) des ersten Befestigungselementes (16) benachbarten Oberfläche der Elektrodenleitung (10).

9. Elektrodenanordnung nach Anspruch 8, **dadurch gekennzeichnet, dass** die erste Elektrode (14) von der von dem ersten Befestigungselement (16) umschlungenen Oberfläche der Elektrodenleitung (10) gebildet wird.

10. Elektrodenanordnung nach Anspruch 8 oder 9, **dadurch gekennzeichnet, dass** das erste Befestigungselement (16) elektrisch leitend ausgebildet ist und elektrisch mit der ersten Elektrode (14) derart verbunden, dass die erste Elektrode (14) und das erste Befestigungselement (16) gemeinsam als eine Elektrode zur Abgabe elektrischer Impulse oder Aufnahme elektrischer Signale wirken.

11. Elektrodenanordnung nach einem der Ansprüche 8 bis 10, **dadurch gekennzeichnet, dass** das erste Befestigungselement (16) im Bereich des distalen Endes der ersten Elektrode (14) an der Elektrodenleitung (10) befestigt ist, während sich das erste freie Ende (40) des ersten Befestigungselementes (16) in der Nähe des proximalen Endes der ersten Elektrode (14) befindet.

12. Elektrodenanordnung nach einem der Ansprüche 1 bis 11, **gekennzeichnet durch** eine am distalen Ende (12) der Elektrodenleitung (10) angeordnete zweite Elektrode (20).

13. Elektrodenanordnung nach einem der Ansprüche 1 bis 12, **gekennzeichnet durch** genau zwei Befestigungselemente (16, 22), welche als korkenzieherartige Helixwendeln mit jeweils einem freien, zum Eindringen in Gewebe ausgebildeten Ende ausgebildet und zum einen am distalen Ende (12) der Elektrodenleitung (10) und zum anderen entfernt hiervon angeordnet sind, sowie **durch** mindestens zwei Elektroden (14, 20) mit elektrisch leitender Oberfläche, die jeweils in unmittelbarer Nachbarschaft zu den Befestigungsmitteln angeordnet sind.

14. Elektrodenanordnung nach einem der Ansprüche 11 und 12 oder 13 **dadurch kennzeichnet, dass** die Elektrodenleitung (10) zwei koaxiale, elektrisch leitende Wendeln umfasst, welche zueinander elektrisch isoliert sind und von denen die äußere Wendel die vom distalen Ende der Elektrodenleitung (10) entfernte erste Elektrode kontaktiert, während die innere Wendel die Elektrode am distalen Ende der Elektrodenleitung (10) kontaktiert.

15. Elektrodenanordnung nach einem der Ansprüche 1 bis 14, **gekennzeichnet durch** Steuermittel im Inneren der Elektrodenleitung 10, welche derart ausgebildet sind, dass das distale Ende (12) der Elektrodenleitung (10) vom proximalen Ende der Elektrodenleitung (10) her in eine wählbare Richtung lateral auslenkbar ist.

## Claims

1. Electrode arrangement comprising an electrode line (10), which carries at least one fixing element (16) and a second fixing element (22), which are constructed for fixing the electrode line to body tissue, in particular the myocardium of a heart, the first fixing element (16) being arranged with spacing from the distal end (12) of the electrode line (10) and the second fixing element (22) being arranged at the distal end (12) of the electrode line (10), **characterised in that** the first fixing element comprises at least one portion that is wound in the manner of a helical coil and is rigidly connected to the electrode line (10), so the helically wound portion substantially coaxially wraps around the electrode line (10) with spacing from the electrode line (10) and comprises a free end that points in the proximal direction, comprises an extension component in the tangential direction with respect to the electrode line (10) and is constructed such that, on insertion, a rotational movement of the electrode line (10) about its longitudinal axis causes the first fixing element (16) to penetrate adjacent body tissue, and **in that** the second fixing element (22) is constructed in the manner of a corkscrew as a helical coil with a free end (40) and the direction of rotation of the second fixing element (22) is counter to that of the first fixing element.

2. Electrode arrangement according to claim 1, **characterised in that** the turns of the coil of the portion wound in the manner of a helical coil of the first fastening element (16) are set apart from one another in the longitudinal direction of the electrode line (10).

3. Electrode arrangement according to either claim 1 or claim 2, **characterised in that** the free first end of the first fixing element (40) is pointed.

4. Electrode arrangement according to any one of claims 1 to 3, **characterised in that** the first fixing element (16) comprises a second free end, which, based on the electrode line (10), has the same tangential alignment as the first free end but an opposing axial alignment, and **in that** the first fixing element (16) is fixed between both free ends to the electrode line (10).

5. Electrode arrangement according to any one of claims 1 to 4, **characterised in that** the second fixing element (22) is connected to the electrode line (10) such that, together with the electrode line, it may be driven in rotation by rotating the electrode line about the longitudinal axis thereof.

6. Electrode arrangement according to claim 4, **characterised by** a drive, which is connected to the second fixing element (22) and is constructed such that the second fixing element (22) may be driven in rotation independently of the electrode line (10) about the longitudinal axis thereof.

7. Electrode arrangement according to claim 6, **characterised in that**, viewed from the proximal end of the electrode line (10), the drive is constructed such that the second fixing element (22) may be driven in rotation relative to the rest of the electrode line (10).

8. Electrode arrangement according to any one of claims 1 to 7, **characterised by** a first electrode (14) comprising an electrically conductive surface on the surface of the electrode line (10) that is adjacent to the free end (40) of the first fixing element (16).

9. Electrode arrangement according to claim 8, **characterised in that** the first electrode (14) is formed by the surface, around which the first fixing element (16) is wound, of the electrode line (10).

10. Electrode arrangement according to either claim 8 or claim 9, **characterised in that** the first fixing element (16) is electrically conductive and electrically connected to the first electrode (14) such that the first electrode (14) and the first fixing element (16) jointly act as an electrode for issuing electrical pulses or picking up electrical signals.

11. Electrode arrange according to any one of claims 8 to 10, **characterised in that** the first fixing element (16) is fixed to the electrode line (10) in the region of the distal end of the first electrode (14), while the first free end (40) of the first fixing element (16) is located in proximity to the proximal end of the first electrode (14).

12. Electrode arrangement according to any one of claims 1 to 11, **characterised by** a second electrode (20) arranged at the distal end (12) of the electrode line (10).

13. Electrode arrangement according to any one of claims 1 to 12, **characterised by** precisely two fixing elements (16, 22), which are constructed as corkscrew-like helical coils, each with a free end constructed for penetrating tissue, and are arranged, on the one hand, at the distal end (12) of the electrode line (10) and, on the other hand, remote therefrom, and by at least two electrodes (14, 20) having an electrically conductive surface, each of which is arranged in immediate proximity to the fixing means.

14. Electrode arrangement according to any one of claims 11 and 12 or 13, **characterised in that** the electrode line (10) comprises two coaxial, electrically conductive coils, which are electrically insulated relative to each other, and of which the outer coil contacts the first electrode, which is remote from the distal end of the electrode line (10), while the inner coil contacts the electrode at the distal end of the electrode line (10).

15. Electrode arrangement according to any one of claims 1 to 14, **characterised by** control means inside the electrode line (10), which are constructed such that the distal end (12) of the electrode line (10), viewed from the proximal end of the electrode line (10), may be laterally deflected in a selectable direction.

## Revendications

1. Dispositif d'électrode équipé d'un fil d'électrode (10), qui comporte au moins un premier élément de fixation (16) et un second élément de fixation (22), qui sont réalisés en vue de la fixation d'un fil d'électrode à un tissu corporel, en particulier au myocarde du coeur,
dans lequel le premier élément de fixation (16) est disposé éloigné de l'extrémité distale (12) du fil d'électrode (10) et le second élément de fixation (22) est disposé à l'extrémité distale (12) du fil d'électrode (10),
**caractérisé**
**en ce que** le premier élément de fixation (16) présente au moins un tronçon enroulé hélicoidalement, qui est relié de façon rigide au fil d'électrode (10), de sorte que le tronçon enroulé hélicoïdalement entoure le fil d'électrode (10) de façon sensiblement coaxiale éloigné du fil d'électrode (10) et présente une extrémité libre allant dans une direction proximale, qui présente un composant longitudinal par rapport à la direction tangentielle du fil d'électrode (10) et est réalisée de sorte que le premier élément de fixation (16) pénètre le tissu corporel voisin par un mouvement de rotation du fil d'électrode (10) autour de son axe longitudinal selon l'application,
et **en ce que** le second élément de fixation (22) est réalisé sous la forme d'une hélice avec une extrémité libre (40) selon la technique du tire-bouchon et le sens de rotation du second élément de fixation (22) est inversé par rapport à celui du premier élément de fixation.

2. Dispositif d'électrode selon la revendication 1, **caractérisé en ce que** les spires de l'hélice du tronçon enroulé en hélice du premier élément de fixation (16) sont éloignées les unes des autres dans la direction longitudinale du fil d'électrode (10).

3. Dispositif d'électrode selon l'une des revendications 1 à 2, **caractérisé en ce que** la première extrémité libre du premier élément de fixation (40) est affûtée.

4. Dispositif d'électrode selon l'une des revendications 1 à 3, **caractérisé en ce que** le premier élément de fixation (16) présente une seconde extrémité libre, laquelle possède la même direction tangentielle que la première extrémité libre par rapport au fil d'électrode (10) mais une direction axiale inversée, et **en ce que** le premier élément de fixation (16) est fixé entre deux extrémités libres au fil d'électrode (10).

5. Dispositif d'électrode selon l'une des revendications 1 à 4, **caractérisé en ce que** le second élément de fixation (22) est relié au fil d'électrode (10) de sorte qu'il est entraîné en rotation conjointement avec le fil d'électrode par rotation du fil d'électrode autour de son axe longitudinal.

6. Dispositif d'électrode selon la revendication 4, **caractérisé par** un entraînement, qui est relié au second élément de fixation (22) et est réalisé de sorte que le second élément de fixation (22) peut être entraîné à rotation indépendamment du fil d'électrode (10) autour de son axe longitudinal.

7. Dispositif d'électrode selon la revendication 6, **caractérisé en ce que** l'entraînement est réalisé de sorte que le second élément de fixation (22) peut être entraîné à rotation depuis l'extrémité proximale du fil d'électrode (10) par rapport au reste du fil d'électrode (10).

8. Dispositif d'électrode selon l'une des revendications 1 à 7, **caractérisé par** une première électrode (14) munie d'une surface électriquement conductrice sur la surface adjacente à l'extrémité libre (40) du premier élément de fixation (16) du fil d'électrode (10).

9. Dispositif d'électrode selon la revendication 8, **caractérisé en ce que** la première électrode (14) est formée par la surface entourant le premier élément de fixation (16) du fil d'électrode (10).

10. Dispositif d'électrode selon la revendication 8 ou 9, **caractérisé en ce que** le premier élément de fixation (16) est réalisé électriquement conducteur et est relié électriquement à la première électrode (14) de sorte que la première électrode (14) et le premier élément de fixation (16) fonctionnent conjointement comme une électrode destinée à délivrer des impulsions électriques ou à recevoir des signaux électriques.

11. Dispositif d'électrode selon l'une des revendications 8 à 10, **caractérisé en ce que** le premier élément de fixation (16) est fixé au fil d'électrode (10) dans la zone de l'extrémité distale de la première électrode (14), tandis que la première extrémité libre (40) du premier élément de fixation (16) se trouve dans le voisinage de l'extrémité proximale de la première électrode (14).

12. Dispositif d'électrode selon l'une des revendications 1 à 11, **caractérisé par** une seconde électrode (20) disposée à l'extrémité distale (12) du fil d'électrode (10).

13. Dispositif d'électrode selon l'une des revendications 1 à 12, **caractérisé par** exactement deux éléments de fixation (16, 22), qui sont réalisés sous la forme d'hélices selon la technique du tire-bouchon avec respectivement une extrémité libre réalisée en vue de pénétrer dans un tissu et sont disposés d'une part à l'extrémité distale (12) du fil d'électrode (10) et d'autre part éloignés de celle-ci, ainsi que par au moins deux électrodes (14, 20) avec une surface électriquement conductrice, qui sont disposées respectivement au voisinage immédiat des moyens de fixation.

14. Dispositif d'électrode selon l'une des revendications 11 et 12 ou 13, **caractérisé en ce que** le fil d'électrode (10) comprend deux hélices coaxiales électriquement conductrices, qui sont électriquement isolées l'une de l'autre et grâce auxquelles l'hélice externe vient en contact de la première électrode éloignée de l'extrémité distale du fil d'électrode (10), tandis que l'hélice interne vient en contact de l'électrode à l'extrémité distale du fil d'électrode (10).

15. Dispositif d'électrode selon l'une des revendications 1 à 14, **caractérisé par** des moyens de commande à l'intérieur du fil d'électrode (10), qui sont réalisés de telle sorte que l'extrémité distale (12) du fil d'électrode (10) est déviée de l'extrémité proximale du fil d'électrode (10) latéralement dans une direction au choix.
